# FASCICULE DE BREVET EUROPEEN

(11) **EP 3 804 013 B1**
(45) Date de publication et mention de la délivrance du brevet: **15.02.2023**
(21) Numéro de dépôt: 19740647.3
(22) Date de dépôt: 31.05.2019
(51) Int. Cl.: H01M 4/86, H01M 4/90, H01M 8/04082, H01M 8/1025, H01M 8/16

(54) **BIOPILE A RESERVOIR DE COMBUSTIBLE**
BIOZELLE MIT BRENNSTOFFRESERVOIR
BIOCELL WITH FUEL RESERVOIR

(30) Priorité: 08.06.2018 FR 1855014
(43) Date de publication de la demande: 14.04.2021
(73) Titulaire: Centre national de la recherche scientifique, 75016 Paris (FR); Université Grenoble Alpes, 38400 Saint-Martin-d'Hères (FR); Ecole Centrale de Lyon, 69130 Ecully (FR); UNIVERSITE CLAUDE BERNARD - LYON 1, 69100 Villeurbanne (FR)
(72) Inventeur: LE GOFF, Alan, 38830 Crêts-en-Belledonne (FR); NEDELLEC, Yannig, 38000 Grenoble (FR); ABREU, Caroline, 89100 Sens (FR); COSNIER, Serge, 38920 Crolles (FR); HOLZINGER, Michaël, 38830 Saint-Laurent-du-Pont (FR)
(74) Mandataire: LLR
(86) Numéro de dépôt international: PCT/IB2019/054544
(87) Numéro de publication internationale: WO 2019/234573

(56) Documents cités:
- US-A1- 2013 280 509
- US-A1- 2015 280 266
- CAROLINE ABREU ET AL: "Assembly and Stacking of Flow-through Enzymatic Bioelectrodes for High Power Glucose Fuel Cells", ACS APPLIED MATERIALS & INTERFACES, vol. 9, no. 28, 7 juillet 2017 (2017-07-07), pages 23836-23842, XP055542906, US ISSN: 1944-8244, DOI: 10.1021/acsami.7b06717

## Description

### Domaine de l'invention

L'invention se rapporte à une pile enzymatique, ou biopile, à combustible, à ses utilisations pour la détection ou l'oxydation d'un biocombustible tel que le glucose, à des kits la comprenant ainsi qu'à des appareils électriques ou électroniques l'incorporant. L'invention porte également sur des procédés de fabrication de cette biopile ainsi qu'à des assemblages comprenant au moins deux biopiles selon l'invention.

### Art antérieur

La technologie des piles à combustible se base sur la conversion de l'énergie chimique en énergie électronique. Une molécule organique telle que le glucose est une des sources d'énergie les plus importantes de nombreux organismes vivants et peut être considérée comme un biocombustible sûr, facile à manipuler, biodégradable puisque consommable.

Les piles enzymatiques (également appelées biopiles) à biocombustible utilisent des enzymes pour produire de l'énergie ou de la puissance électrique à partir de substrats biologiques tels que l'oxygène, le méthanol, le glucose ou l'amidon. Les biopiles à combustible convertissent le biocombustible en présence de composés enzymatiques ce qui produit de la puissance. Les biopiles les plus connues fonctionnent par oxydation du glucose (GBFC) sont des piles de ce type qui convertissent le glucose par oxydation à l'anode pour la production de puissance en utilisant une enzyme incorporée à celle-ci et ayant une fonction de catalyseur de la réaction. La cathode a généralement pour fonction de réduire l'oxygène et peut, ou non, comprendre une enzyme catalysant cette réaction.

Les enzymes sont des alternatives prometteuses à des catalyseurs à base de métaux nobles puisque la plupart d'entre elles sont opérationnelles à pH neutre et à température ambiante et offre une toxicité faible ou nulle, ce qui n'est pas le cas d'autres catalyseurs à base de métaux. Ainsi de telles biopiles à biocombustible peuvent être très facilement recyclées et présenter des alternatives écologiques en tant que générateur de courant électrique en particulier pour des dispositifs jetables.

CAROLINE ABREU ET AL: "Assembly and Stacking of Flow-through Enzymatic Bioelectrodes for High Power Glucose Fuel Cells", ACS APPLIED MATERIALS & INTERFACES, vol. 9, no. 28, 7juillet 2017, pages 2383623842, décrit une pile à combustible comprenant une feuille en cellulose séparant les électrodes. Cette feuille en cellulose ne présente qu'une masse surfacique faible de 64 g/m².

La présente invention concerne plus particulièrement la fabrication d'une biopile de type «bouton», c'est-à-dire de faible dimension et capable de fournir une électricité équivalente à une pile bouton à base de métaux et pouvant éventuellement être jetée à la poubelle sans risque pour l'environnement. Ceci n'est pas le cas des piles boutons et des piles alcalines à l'heure actuelle. Cette technologie verte pourrait fournir une solution originale aux problèmes liés au recyclage des piles à base de zinc, lithium ou de manganèse utilisées au quotidien. Sans présence de métaux, le dispositif selon l'invention décrit également une source d'énergie portative invisible aux détecteurs de métaux.

Alternativement ou additionnellement, le dispositif selon l'invention vise à permettre la production d'une énergie électrique pour un besoin ponctuel lorsque d'autres sources d'énergie conventionnelles (éolienne, solaire, etc...) ne sont pas disponibles. En effet, les enzymes ont une sélectivité unique qui permet la production de puissance dans des milieux complexes ce qui peut permettre l'utilisation de liquide et en particulier de liquides biologiques divers : sucre (glucose), boissons sucrées, fluides physiologiques (salive, sang, urine), d'origine animale ou végétale (jus de fruits) etc.) en tant qu'activateur et/ou de combustible. Dans ce contexte le terme « combustible » et « biocombustible » est interchangeable.

A ces égards, d'importants défis sont à surmonter avant de pouvoir mettre en pratique commerciale de telles biopiles.

En particulier, elles doivent, de préférence, être de petite (1 à 5 cm² de surface), voire de très petite (moins de 0,5 cm² de surface) taille, et être conçues pour pouvoir remplacer les piles de types «bouton» fréquemment utilisées dans des dispositifs jetables. Elles doivent avantageusement être aisément recyclables, et de préférence peu coûteuses.

Ainsi, l'invention vise notamment à résoudre le problème de la fourniture d'une biopile à combustible, en particulier de conception permettant son utilisation dans des dispositifs jetables, peu coûteuse (type piles boutons) ou à usage unique, de préférence de dimensions restreintes, et permettant avantageusement un accès optimisé du combustible et de l'oxydant à la cellule électrochimique, pour produire le maximum d'énergie. De plus il est avantageux que cette biopile à combustible puisse être utilisée en un assemblage de plusieurs biopiles à biocombustibles ou cellules électrochimiques, pour produire une énergie durable et renouvelée.

Ainsi, d'une manière générale, l'invention vise à résoudre le problème de la fabrication aisée et à faibles coûts des biopiles à combustible et leur intégration dans des circuits électriques plus ou moins sophistiqués dans lesquels les densités de courant produites sont suffisamment élevées pour faire fonctionner lesdits dispositifs. Avantageusement, la tension délivrée est suffisante pour faire fonctionner les dispositifs de manière fiable.

### Description de l'invention

Selon un mode de réalisation l'invention comprend une biopile à réservoir de biocombustible, étant destinée à être mise en contact avec un milieu liquide, ledit milieu liquide comprenant éventuellement un biocombustible, et un milieu fluide comprenant un oxydant, ladite biopile comprenant une première cellule électrochimique, ladite première cellule électrochimique comprenant :
- une anode constituée d'un agglomérat solide comprenant un matériau conducteur mélangé à une première enzyme apte à catalyser l'oxydation du biocombustible ; et
- une cathode constituée d'un agglomérat solide comprenant un matériau conducteur mélangé à une seconde enzyme apte à catalyser la réduction de l'oxydant, et
- une membrane séparatrice et poreuse, électriquement isolante, et perméable audit milieu liquide, placée entre l'anode et la cathode.

Ladite biopile comprend, en outre, des moyens de mise en circuit électrique de ladite biopile avec un récepteur électrique, lesdits moyens de mise en circuit électrique permettant la circulation du courant entre l'anode et la cathode. La biopile est caractérisée en ce qu'elle comprend un moyen de stockage du biocombustible et de mise à disposition du milieu liquide à l'anode, ledit moyen comprenant un matériau poreux hydrophile en contact avec ladite anode et présentant une masse surfacique de 500 à 900g/m².

Le moyen de stockage peut donc agir en tant que réservoir de biocombustible et/ou d'un liquide le contenant. De préférence le moyen de stockage présente une masse surfacique (ou grammage) pouvant aller de 650 à 750 g/m² de manière à être suffisamment absorbant. Avantageusement sa masse surfacique est de 703 g/m², éventuellement ± 20%. Elle peut en particulier être choisie dans le groupe de valeurs constitué par 655, 660, 665, 670, 675, 680, 685, 690, 695, 696, 697, 698, 699, 700, 701, 702, 703, 704, 705, 707, 708, 709, 710, 711, 712, 713, 714, 715, 720, 725, 730, 735, 740, 745, 750, 755 et 760 g/m². Ainsi le moyen de stockage est un matériau très poreux.

Le moyen de stockage peut être sous la forme d'un coussinet poreux, d'un tampon ou, en anglais « pad ». Le matériau constitutif du moyen de stockage est de préférence un isolant électrique. Ce matériau peut comprendre ou être constitué de fibres tissées, ou non tissées (feutre). Ces fibres sont de préférence des fibres naturelles et/ou biodégradables, par exemple il peut s'agir de matériau comprenant, ou étant essentiellement constitué de, ou encore à base de cellulose, et en particulier de coton. Il peut également s'agir d'un polymère naturel (cellulose, coton, éponge, chitosan etc...) ou synthétique (mousse polyacrylique, polyvinyle alcool, etc...). L'expression « à base de X» peut être interprétée comme visant un matériau constitué de plus de 90% en masse, de préférence de plus de 95%, voire de plus de 98% par rapport à la masse totale du constituant X. Le moyen de stockage peut donc avantageusement comprendre, ou être essentiellement constitué, d'une feuille de papier, de préférence de type buvard, par exemple de buvard super-épais. Il est également préféré que ce coussinet et/ou la feuille de papier soit sans additifs. Le grammage peut être mesuré selon la norme NF EN ISO 536 Novembre 2012.

L'épaisseur du moyen de stockage est avantageusement aussi faible que possible pour permettre une utilisation miniaturisée. Cependant, pour d'autres utilisations, cette épaisseur peut être plus conséquente et de l'ordre même du centimètre, ou du demi centimètre. Cette épaisseur peut donc être de 1 cm à 0,1 mm, de préférence de 8 à 2 mm, et plus particulièrement aux environs de 2,6mm, éventuellement ± 20%. Elle peut en particulier être sélectionnée comme étant une épaisseur choisie dans le groupe constitué par 1 ; 1,5 ; 1,6 ; 1,7 ; 1,8 ; 1,9 ; 2 ; 2,1 ; 2,2 ; 2,3 ; 2,4 ; 2,6 ; 2,7 ; 2,8 ; 2,9 ; 3 ; 3,1 ; 3,2 ; 3,3 ; 3,4 ; 3,5 ; 3,6 ; 3,7 ; 3,8 ; 3,9 et 4 mm.

Avantageusement le moyen de stockage est une feuille de papier buvard, d'une épaisseur de 2,6 mm ± 0.2 mm. Sa porosité doit permettre une bonne absorption du liquide contenant le combustible (par exemple eau, urine, boisson sucrée, sang, etc....) et en particulier le sucre. Selon une variante préférée de l'invention il doit être suffisamment rigide pour l'enchâssement ou le maintien de bioanodes ou de biocathodes.

Avantageusement le moyen de stockage présente un temps de filtration du liquide pouvant aller de 100 à 140 s/ml, de préférence de 110 à 130 s/ml et avantageusement aux alentours de 120s/100 ml, éventuellement ± 20% ; mesuré selon la méthode de Herzberg (avec une colonne d'eau de 100 mm).

La cellule électrochimique comprise dans la biopile selon l'invention comprend une anode et une cathode. Ces électrodes sont sous forme d'un agglomérat solide qui comprend à sa base un matériau conducteur de préférence poreux et au moins une enzyme de la demi-réaction à catalyser. Ce matériau poreux peut être tout matériau conducteur poreux recyclable tel que feutre de carbone, carbone microporeux, nanotubes de carbone, charbon actif, noir de carbone, polymères conducteurs, etc. Dans les exemples, des pastilles à base de nanotubes de carbone à parois simples ou plus avantageusement à multi-paroi (MWCNT) ou de noir de carbone offrent une excellente porosité associée à une excellente conductivité. Par « nanotube de carbone », on entend un nanotube de carbone dont au moins une dimension est inférieure à 1500 nm. De préférence, les nanotubes de carbone ont un rapport longueur (L) sur diamètre noté L/diamètre compris entre 100 et 5000. De préférence les nanotubes de carbone ont une longueur d'environ 1,5 µm et par exemple un diamètre d'environ 10 nm.

Dans les exemples de réalisation de l'invention de la demande le biocombustible choisi est le glucose, du fait de la grande disponibilité de ce composé et de son peu d'impact sur l'environnement. Cependant la structure de la biopile selon l'invention peut s'adapter à des substrats autres que le glucose dans la mesure où les composés enzymatiques associés sont également adaptés.

Le bilan réactionnel théorique de la biopile enzymatique glucose/O2 est le suivant:

Anode: glucose → gluconolactone + 2 H⁺ + 2 e⁻

Cathode: O2 + 4 H⁺ + 4 e⁻ → 2 H2O

Biopile: 2 glucose + O2 → 2 gluconolactone + 2 H2O

Ainsi selon un aspect préféré de l'invention un système enzymatique utilisé à l'anode peut comprendre au moins une glucose oxydase. Les glucoses oxydases (GOx) sont des enzymes oxydo-réductases du type EC 1.1.3.4 (classification avril 2018) qui catalysent l'oxydation du glucose, plus particulièrement du β-D-glucose (ou Dextrose) en peroxyde d'hydrogène et en D-glucono-δ-lactone, qui ensuite s'hydrolyse en acide gluconique. Les glucoses oxydases se lient spécifiquement au β-D-glucopyranose (forme hémiacétal du glucose) et n'agissent pas sur l'α-D-glucose. Elles sont cependant capables d'agir sur le glucose sous ses formes énantiomèriques, car en solution le glucose adopte principalement sa forme cyclique (à pH7: 36.4 % de α-D-glucose et 63,6 % de β-D-glucose, 0,5 % sous forme linéaire). De plus l'oxydation et la consommation de la forme β déplace l'équilibre α-D-glucose/β-D-glucose vers cette forme. Le terme GOx s'étend aux protéines natives et à leurs dérivés, mutants et/ou équivalents fonctionnels. Ce terme s'étend en particulier aux protéines qui ne diffèrent pas de manière substantielle au niveau de la structure et/ou de l'activité enzymatique.

Les glucoses oxydases comprennent et requièrent un cofacteur pour permettre la catalyse. Ce co-facteur est la Flavine Adénine Dinucléotide (FAD), un composant majeur d'oxydation-réduction dans les cellules. La FAD sert d'accepteur d'électron initial, elle est réduite en FADH₂ qui sera ré-oxydée en FAD (régénération) par l'oxygène moléculaire (O₂, plus réducteur que le FAD). L'O₂ est enfin réduit en peroxyde d'hydrogène (H₂O₂). Le cofacteur est compris dans l'enzyme GOx disponible dans le commerce et le terme GOx et FAD-GOX est équivalent.

La glucose oxydase la plus utilisée est celle extraite d'*Aspergillus niger.* Cependant de la GOx d'autres sources peut être utilisée, comme par exemple certaines souches de l'espèce *Penicillium* ou d*'Aspergillus terreus.*

La glucose oxydase *d'Aspergillus niger* est un dimère composé de 2 sous-unités égales avec un poids moléculaire de 80 kDa chacune (par gel filtration). Chaque sous-unité contient une flavine adénine dinucléotide et un atome de fer. Cette glycoprotéine contient environ 16% de sucre neutre et 2% de sucres aminés. Elle contient également 3 résidus de cystéine et 8 sites potentiels pour la N-glycosylation.

L'activité spécifique de la GOx est de préférence supérieure ou égale à 100,000 unités/g solide (sans ajout d'O₂). Une unité est définie comme la capacité d'oxydation de 1,0 µmole de β-D-glucose en D-gluconolactone et d'H2O2 par minute à pH 5.1 à 35 °C. (Km = 33-110 mM; 25 °C; pH 5,5 - 5,6).

Dans la mesure où l'utilisation de la GOx implique la production d'eau oxygénée on peut ajouter au système enzymatique de la catalase.

La catalase est une enzyme tétramère catalysant la réaction : 2 H₂O₂ → O₂ + 2 H₂O. Chaque sous-unité contient du fer lié à un groupe protohème de type IX. Chaque sous-unité est équivalente et comprends une chaine polypeptidique d'environs 500 acides aminés. Le poids moléculaire de chaque sous-unité est généralement de 60 kDa (gel filtration). La catalase peut se lier fortement à la NADP et celle-ci et le groupe hémique se trouvent alors positionnés à 13.7 Å l'un de l'autre. Elle peut réagir avec d'autres peroxydes alkyl hydrogénés tels que le méthyl peroxyde ou l'éthyle peroxyde. L'activité de la catalase est généralement constante dans un domaine de pH allant de 4 à 8,5. Son activité spécifique est, de préférence, supérieure à 2000 unités/mg, notamment supérieure à 3000 unité /mg par exemple environ 5000 unités/mg de protéines. Une unité est définie comme la capacité de décomposer 1.0 micromole d'eau oxygénée (H₂O₂) par minute à pH 7.0 à 25°C, la concentration H₂O₂ tombant de préférence de 10,3 à 9,2 millimolaire. Le terme catalase s'étend aux protéines natives et à leurs dérivés, mutants et/ou équivalents fonctionnels. Ce terme s'étend en particulier aux protéines qui ne diffèrent pas de manière substantielle au niveau de la structure et/ou de l'activité enzymatique. La catalase utilisée est de préférence d'origine bovine.

Il est également possible d'utiliser d'autres enzymes transformant le glucose, et particulièrement au moins une déshydrogénase. En effet l'eau oxygénée n'est pas produite lors de la réaction catalysée par cette enzyme ce qui est avantageux. Les déshydrogénases fonctionnent également en association avec de la FAD (cf. supra). Une déshydrogénase particulièrement préférée est la Flavine Adénine Dinucléotide - Glucose DésHydrogénase (FAD-GDH) (EC 1.1.5.9). Le terme FAD-GDH s'étend aux protéines natives et à leurs dérivés, mutants et/ou équivalents fonctionnels. Ce terme s'étend en particulier aux protéines qui ne diffèrent pas de manière substantielle au niveau de la structure et/ou de l'activité enzymatique. Ainsi on peut utiliser pour réaliser l'anode de la cellule électrochimique de la biopile selon l'invention, en association avec un cofacteur, une protéine enzymatique GDH présentant une séquence d'acides aminés possédant au moins 75%, de préférence 95%, et encore plus préférentiellement 99% d'identité avec la ou les séquences GDH telles que répertoriées dans les banques de données (par exemple SWISS PROT). Une FAD-GDH *d'aspergillus sp.* est particulièrement préférée et efficace mais d'autres FAD-GDH provenant de *Glomerella cingulata* (GcGDH), ou une forme recombinante exprimée dans *Pichia pastoris* (rGcGDH), pourraient également être utilisées. La FAD-GDH utilisée dans un mode de réalisation exemplifié est une FAD-GDH *d'aspergillus sp.* (SEKISUI DIAGNOSTICS, Lexington, MA, No. Catalogue GLDE - 70 - 1192) qui présente les caractéristiques suivantes :
Aspect : poudre jaune lyophilisée.
Activité : > 900 U/mg poudre 37°C.
Solubilité : se dissout aisément dans l'eau à une concentration de : 10mg/mL.
Une unité d'activité : quantité d'enzyme qui va convertir une micromole de glucose par minute à 37°C.
Poids moléculaire (Gel Filtration) 130kDa.
Poids moléculaire (SDS Page) : bande diffuse à 97 kDa indicative d'une protéine glycosylée.
Point isoélectrique : 4,4.
Valeur Kₘ : 5.10⁻² M (D-Glucose).

Le matériau conducteur poreux peut également comprendre une molécule aromatique agissant en tant que médiateur redox, telle que 1,4-naphtoquinone, pour améliorer les échanges électroniques. D'autres molécules choisies dans le groupe formé par la 9,10-phénanthrènequinone, la 1,10-phénanthroline-5,6-dione, la 9,10-anthraquinone, le phénanthrène, la 1,10-phénanthroline, la 5-méthyl-1,10-phénanthroline, le pyrène, le 1-aminopyrène, l'acide pyrène-1-butyrique, et les mélanges de deux ou plus de ceux-ci peuvent également être considérées. L'utilisation de tels composés se révèlent particulièrement avantageux dans le cas de systèmes enzymatiques comprenant une FAD-GDH ou une GOx.

L'oxydant de la biopile peut avantageusement être de l'oxygène moléculaire, et en particulier de l'oxygène contenu dans l'air.

Lorsque l'oxydant est de l'oxygène moléculaire O₂, le système enzymatique qui peut être utilisé à la cathode peut avantageusement comprendre une bilirubine oxydase (BOD). La BOD est une enzyme oxydoréductase (Classification EC 1.3.3.5, numéro CAS 80619-01-8 ; avril 2018) catalysant la réaction :

2 bilirubine + O(2) <=> 2 biliverdine + 2 H(2)O.

La bilirubine oxydase la plus utilisée est celle extraite de *Myrothecium verrucaria.* Cependant l'utilisation de BOD d'autres sources peut être considérée. L'activité de la BOD est avantageusement supérieure à 15 unité/mg de protéine, de préférence supérieure à 50 unité/mg, par exemple aux environs de 65 unité/mg de protéine. Une unité est définie comme la capacité d'oxyder 1,0 micromole de bilirubine par minute à pH 8,4 à 37°C. Le terme BOD s'étend aux protéines natives et à leurs dérivés, mutants et/ou équivalents fonctionnels. Ce terme s'étend en particulier aux protéines qui ne diffèrent pas de manière substantielle au niveau de la structure et/ou de l'activité enzymatique.

La protoporphyrine IX (numéro CAS 553-12-8 ; avril 2018), est un composé à motif porphyrinique de formule brute C₃₄H₃₄N₄O₄. Elle est utilisée pour fonctionnaliser le matériau conducteur poreux, et en particulier les nanotubes, et permettre une meilleure orientation d'enzymes tels que des BODs. Elle est donc avantageusement comprise dans le matériau constituant la cathode.

L'agglomérat solide qui combine un matériau conducteur poreux et au moins une enzyme et/ou un système enzymatique se présente de préférence sous forme de bloc ou de pastille, par exemple circulaire, avantageusement obtenu par compression du mélange des éléments. L'agglomérat peut être obtenu aisément par compression et prendre toute forme particulière souhaitée. En particulier, les bioanodes et/ou biocathodes selon l'invention peuvent prendre la forme de petite (1 à 2 cm de diamètre), voire de très petite (moins de 0.5 cm de diamètre), pastilles, par exemple circulaires ou polygonales. De telles pastilles peuvent avoir une épaisseur variant de 3,5 mm à 0,2 mm, par exemple 0,25 mm. De ce fait la biopile selon l'invention peut être de forme variée et de petite dimension. Notamment elle peut n'occuper qu'un volume inférieur ou égal à 2 cm³, de préférence inférieur ou égale à 1 cm³, voire inférieur ou égal à 0.75 cm³. Elle peut notamment être conçue pour pouvoir remplacer les piles de « types boutons ».

Selon un aspect particulièrement préféré de l'invention, l'anode comprend donc une enzyme GOx, de préférence associée à une catalase, ou une enzyme FAD-GDH. Dans ce cas le biocombustible est donc du glucose. Dans les deux cas, la bioanode comprend également un médiateur d'oxydation du glucose, par exemple un composé de type 1,4-naphtoquinone. De préférence la biocathode comprend une enzyme réduisant l'oxygène, et plus particulièrement la BOD, avantageusement associé à de la protoporphyrine IX. Les termes de biocathode et de bioanode réfèrent à la présence de matériel biologique, par exemple une enzyme, dans leur structure. Dans le contexte de la biopile de l'invention ils sont à utiliser de manière équivalente à cathode et anode.

Le dispositif selon l'invention comprend une membrane séparatrice et poreuse, électriquement isolante, et perméable au milieu liquide, qui est placée entre l'anode et la cathode. Cette membrane permet le passage notamment des espèces ioniques entre l'anode et la cathode.

Selon une variante particulière de l'invention, cette membrane peut être une fine feuille (moins de 200 µm d'épaisseur) de papier, qui est de faible masse surfacique (par exemple inférieure ou égale à 150 g/m². Notamment une telle membrane présente une épaisseur de moins de 150 µm, préférablement de moins de 100 µm, préférablement moins de 75 µm de papier, qui est avantageusement biodégradable. Cette option est particulièrement préférée lorsque le moyen de stockage du biocombustible et de mise à disposition du milieu liquide à l'anode encadre et supporte la bioanode et/ou les pastilles anodiques, et éventuellement bio-cathodique(s) de la biopile.

Selon une autre variante préférée de l'invention, la membrane séparatrice et poreuse, électriquement isolante, et perméable au milieu liquide, est également le moyen de stockage du biocombustible et de mise à disposition du liquide. Avantageusement ce moyen de stockage est tel que décrit ci-dessus.

La biopile selon l'invention comprend également des moyens de mise en circuit qui incorpore généralement un matériau conducteur d'électricité. Ces moyens peuvent être sous forme de couches, de languettes ou de fils. Une telle couche, languette ou fil présente avantageusement une faible épaisseur, une haute conductivité thermique et/ou électrique et peut comprendre, ou être (substantiellement) constitué de, graphite hautement orienté. Ainsi on peut utiliser une feuille, ou une languette, en graphite pyrolytique (*pyrolytic graphite sheet*)*.* Son épaisseur peut être choisie comme allant de 10 à 500 µm, de préférence de 17 à 300 µm, et avantageusement de 40 à 100µm. Elle peut être de choisie dans le groupe constitué par des épaisseurs de 10, 17, 25, 40, 50, 70, et 100 µm. Sa conductivité thermique (dans le plan longitudinal de la feuille) peut-être de 500 à 2000 W/(m.K), de préférence de 700 à 1950 W/(m.K) et avantageusement 900 à 1350 W/(m.K). Elle peut être de choisie dans le groupe constitué par des valeurs de conductivités thermiques de 700, 1 000, 1 300, 1 350, 1 600, 1850 et 1950 W/(m.K). Cette couche peut également présenter une conductivité électrique supérieure à 5 000 S/cm, de préférence supérieure ou égale à 8000 S/cm, par exemple aux alentours de 10 000 S/cm. Elle peut cependant présenter une conductivité supérieure, par exemple aux environs de 20 000 S/cm, en particulier si l'épaisseur de la couche est inférieure à 40 µm. Cette couche peut également présenter une résistance à la chaleur, par exemple une résistance à une température de plus de 200°C, avantageusement de plus de 300°C, par exemple d'environ 400°C.

Le dispositif selon l'invention comprend avantageusement une revêtement externe qui peut être une couche, ou un film, protecteur qui recouvre en partie la ou les cellules électrochimiques du dispositif. Celui-ci est de préférence flexible, adhésif, non toxique, chimiquement stable, électriquement isolant, peu sensible aux radiations et/ou a une gamme de température de service large (par exemple de -150°C à 200°C, voire aux environs de 260°C). Ce revêtement, ou film protecteur externe, peut comprendre, ou être (substantiellement) constitué d'un tissu en fibres de verre imprégné d'un matériau relativement inerte comme un matériau polymérique perfluoré de type PTFE (polytétrafluoroéthylène) ou un matériau à base de silicone. Le PTFE peut être du Teflon^{®} de Du Pont de Nemours, du Fluon^{®} de Asahi Glass, de Hostaflon ^{®} de Dyneon. Le film ou revêtement est de préférence imprégné de plus de 50% en poids dudit matériau, avantageusement de 50 à 70%, de préférence de 57 à 64 % par rapport au poids total du film. Son épaisseur peut être de quelque dixièmes, voire centièmes de millimètres. Par exemple, elle peut être choisie dans une gamme allant de 0,03 à 0,50 mm, de préférence de 0,05 à 0,30 mm et de préférence de 0,06 à 0,14 mm, par exemple être de 0,07 mm (NF EN ISO 2286 - 3 décembre 2016). Selon un aspect préféré de l'invention, le revêtement, ou film protecteur, comprend une couche adhésive, de préférence résistante à l'eau, lui permettant d'adhérer à la surface externe de la, ou les, cellule(s) électrochimique(s) de la biopile selon l'invention. Selon un aspect particulier, ce film peut être apposé directement sur une face de l'anode et/ou de la cathode, ou directement sur une partie des moyens de mise en circuits. Selon un autre aspect préféré, ce revêtement externe, qui est de préférence flexible et isolant, comprend une ou plusieurs ouvertures positionnée(s) et dimensionnée(s) de manière à permettre l'accès d'un liquide et/ou d'un gaz à l'anode et/ou la cathode. Cette ouverture peut être prédécoupée dans le revêtement : par exemple elle peut prendre la forme d'une série de petites ouvertures circulaires positionnées en regard de la biocathode. Additionnellement ou alternativement cette ouverture peut être constituée par le fait que le revêtement n'entoure pas totalement la biopile comprenant la, ou les, cellule(s) électrochimique(s) et le moyen de stockage mais laisse une ouverture donnant accès à ces éléments. Ainsi pour un dispositif de forme généralement plane, une ouverture peut être laissée sur la tranche du dispositif.

Selon un aspect avantageux de l'invention, lorsque la bioanode et/ou la biocathode (qui peut être sous forme de pastille ou de bouton), est maintenue dans un cadre, ledit cadre étant soit ledit moyen de stockage du biocombustible et de mise à disposition du milieu liquide, soit un matériau électriquement isolant tel que du PTFE.

Ainsi, selon un premier mode de réalisation, l'anode est insérée dans le moyen de stockage, qui peut être par exemple une feuille en matériau absorbant tel que décrit précédemment. Ce moyen de stockage va donc également agir en tant que cadre et/ou support et maintenir la bioanode et éventuellement la biocathode, en position, et, étant généralement en contact direct avec celle-ci, va la maintenir en contact avec le milieu liquide et le biocombustible contenu dans celui-ci. Selon cette variante de l'invention le dispositif peut comprendre plusieurs cellules électrochimiques du type décrit dans la demande, lesdites cellules électrochimiques étant montées en série. Un tel dispositif est donc constitué d'un empilement de cellules électrochimiques le nombre de cellules n'étant généralement pas supérieur à dix. Par exemple trois ou quatre cellules individuelles peuvent être empilées.

Selon un autre mode de réalisation décrit précédemment, le moyen de stockage est également la membrane de la biopile. Dans ce cas, l'anode (et éventuellement la cathode) peut être insérée dans un cadre, ou gabarit, composé d'un matériau isolant, tel que par exemple du PTFE (polytétrafluoroéthylène). Ce cadre a, de préférence, des ouvertures dimensionnées pour recevoir la ou les bioélectrodes et permet de maintenir et/ou de positionner l'électrode (anode ou cathode). En effet, selon cette variante de l'invention le cadre peut contenir plusieurs électrodes qui peuvent être de type anodique ou cathodique. Les cadres sont dimensionnés pour permettre, lors de la mise en regard de deux cadres, de mettre en regard une anode et une cathode et de créer ainsi plusieurs cellules électrochimiques. Les moyens de mise en circuit sont alors avantageusement positionnés pour connecter ces cellules électrochimiques en série.

Un objet de l'invention est également une méthode de fabrication d'une biopile telle que décrite dans la présente demande. Cette méthode comprend l'utilisation d'une feuille de revêtement externe telle que décrite et comprend l'étape de positionner sur une face interne, préférentiellement adhésive, du revêtement externe les moyens de mise en circuit, au moins une électrode (bioanode ou biocathode) en regard desdits moyens de mise en circuit, les moyens de stockage et éventuellement la membrane. La feuille de revêtement externe est dimensionnée de manière à ce qu'une fois les éléments de la biopile positionnés sur la surface adhésive, une surface libre soit présente sur le pourtour de ces éléments. Cette surface libre est positionnée et dimensionnée pour permettre de solidariser ces éléments entre eux et de constituer la biopile.

Suivant une variante de la méthode selon l'invention, les moyens de mise en circuit sont positionnés dans un premier temps sur la face interne du revêtement puis le cadre (ou gabarit) est superposé. Dans un deuxième temps, un mélange sous forme de poudre ou de pâte constitutif d'une anode ou d'une cathode selon l'invention est positionné à l'intérieur de l'ouverture du cadre et cet assemblage est soumis à une compression *in-situ* pour obtenir un agglomérat solide constitutif de l'anode ou de la cathode. Cette étape peut être répétée pour former l'électrode manquante (cathode ou anode) et les deux revêtements sont assemblés, une fois le moyen de stockage positionné entre ces couches, pour former la biopile selon l'invention. De manière avantageuse ces deux parties sont solidarisées par la présence d'un adhésif sur la partie interne du revêtement externe.

L'invention porte également sur une biopile telle que décrite dans la présente demande et comprenant, en outre, un liquide aqueux, ledit liquide comprenant éventuellement un biocombustible. En effet, le biocombustible peut être déjà présent dans le dispositif sous une forme sèche et/ou solide et/ou non-solubilisée et/ou pouvant migrer vers les sites enzymatiques. Par exemple, il peut être incorporé dans, ou positionné à proximité, des moyens de stockage du combustible. Lorsque de l'eau (pure, ou non), est ajoutée, le biocombustible ainsi présent (par exemple du sucre) est dissout dans le milieu ce qui permet aux échanges électrochimiques d'avoir lieu. Alternativement ou additionnellement, le liquide ajouté comprend le biocombustible. Celui-ci peut être, par exemple, un liquide physiologique tel que du sang, de l'urine ou de la salive ou une boisson alcoolisée ou au glucose.

Un objet de l'invention est également un procédé d'obtention d'une biopile comprenant la mise en présence d'une biopile selon l'invention tel que décrite dans la présente demande avec un liquide, de préférence un liquide aqueux, éventuellement comprenant un biocombustible tel que du glucose, de l'amidon ou de l'éthanol.

Un autre objet de l'invention est un appareil comprenant une biopile selon l'invention, et un récepteur électrique (c'est-à-dire à un appareil qui utilise (reçoit) du courant électrique), ladite biopile étant connectée électriquement audit récepteur électrique. Un tel appareil peut être un test, en particulier un test du liquide biologique : par exemple un test de grossesse ou un test de glycémie. Alternativement ou additionnellement la biopile (et/ou le dispositif) selon l'invention peut être incorporé dans un appareil électronique à affichage électronique et/ou à émission de lumière. Plus généralement l'appareil selon l'invention est de type fonctionnant avec des piles de type boutons utilisant des dérivés métalliques. Un tel appareil selon l'invention peut être avantageusement jetable et/ou biodégradable.

Un autre objet de l'invention est un kit pour la fabrication d'une biopile telle que décrite dans la présente demande et qui comprend une biopile telle que décrite dans la présente demande, associé à des indications de mode d'emploi et éventuellement un contenant comprenant un liquide aqueux tel que décrit précédemment.

Un autre objet de l'invention est l'utilisation d'un papier buvard épais tel que décrit précédemment pour la fabrication d'une biopile ou la fabrication d'un dispositif pour l'obtention d'une biopile, de préférence d'une biopile selon l'invention. De préférence le papier buvard ou absorbant est d'une épaisseur de 2,6 mm ± 0.2 mm.

Un autre objet de l'invention est une utilisation d'une biopile selon l'invention pour la génération d'un courant électrique.

### Description des Figures

La Figure 1 est une vue éclatée partielle d'un dispositif selon l'invention tel que décrit à l'exemple 1.
La Figure 2 représente les courbes de polarisation et de puissance d'une biopile GOx/BOD selon l'exemple 1, réalisée à partir du dispositif de l'exemple 1 et d'une solution de 5 mM de glucose.
La Figure 3 représente les courbes de polarisation et de puissance d'une biopile FAD-GDH/BOD selon l'exemple 1, réalisée à partir du dispositif selon l'invention de la Figure 1 et d'une solution de 5 mM de glucose.
La Figure 4 représente les courbes de polarisation et de puissance d'une biopile GOx/BOD selon la réalisation de l'exemple 2, le combustible étant une solution de 5mM de glucose.
La Figure 5 représente les courbes de polarisation et de puissance d'une biopile GOx/BOD selon la réalisation de l'exemple 2, le combustible étant une solution de 150 mM de glucose.
La Figure 6 représente les courbes de polarisation et de puissance d'une biopile FAD-GDH/BOD sur carbone Vulcan^{®} réalisée selon l'exemple 3, le combustible étant une solution de 150 mM de glucose.
La Figure 7 représente les courbes de polarisation et de puissance d'une biopile FAD-GDH/BOD sur MWCNTs réalisée selon l'exemple 1, le combustible étant une solution de 150 mM de glucose.
La Figure 8 montre la production d'énergie par décharge continue à 6 kQ en µW.h par rapport au temps (minutes) et consommation totale du glucose pour des coussinets imbibés de glucose à 5mM et 150 mM pour la biopile de l'exemple 3.
La Figure 9 montre la production d'énergie par décharge continue à 6 kΩ en µWh par rapport au temps (minutes) et consommation totale du glucose pour des coussinets imbibés de glucose à 150 mM pour la biopile de l'exemple 3 (noir de carbone) et celle de l'exemple 1 FAD-GDH/BOD sur MWCNTs.
La Figure 10 comprend un schéma éclaté partiel de la structure d'une biopile 10 présentée à l'exemple 4 ainsi qu'une vue de face de celle-ci, et sur la gauche une vue de coupe.
La Figure 11 comprend un schéma partiel éclaté de la structure d'une biopile 20 présentée à l'exemple 5 ainsi qu'une vue de face de celle-ci, et sur la gauche, une vue de coupe.
La figure 12 représente les courbes de polarisation et de puissance de la biopile selon l'exemple 4 à t=0, t=7 jours et t= 1 mois pour une solution de glucose à 150 mM.
La Figure 13 montre la production d'énergie par décharge continue à 6 kQ en µWh par rapport au temps (minutes) et consommation totale du glucose pour une solution de glucose à 150 mM pour la biopile de l'exemple 4.
La Figure 14 représente les courbes de polarisation et de puissance de la biopile décrite à l'exemple 5 et à la Figure 11 pour une solution de glucose à 150 mM.
La Figure 15 représente les courbes de polarisation et de puissance de la biopile décrite à l'exemple 6 pour une solution de glucose à 150 mM.

### Exemples

### Réactifs :

La glucose oxydase type VII (GOx) d'*Aspergillus Niger* (Avril 2018 : N°. EC : 1.1.3.4, N°. CAS : 9001-37-0, poids moléculaire : 160 kDa (gel filtration), ≥ 100 000 unité/g) provient de la société Sigma-Aldrich (Code du produit G2133).

La catalase de foie bovin (Avril 2018 : N°. EC : 1.11.1.6, N°. CAS : 9001-05-2, poids moléculaire 250 kDa, 2000 à 5000 unité/mg) provient également de la société Sigma-Aldrich Co. (code du produit C1345).

La bilirubine oxydase (BOD) de *Myrothecium Verrucaria* (avril 2018 EC No. 1.3.3.5, N° CAS : 80619-01-8, 15 à 65 unités/mg) a été obtenue auprès de la société Sigma Aldrich (code du produit B0390).

La protoporphyrine IX pure à 95% provient également de Sigma-Aldrich Co. (code du produit P8293).

La flavine adénine dinucléotide-dépendante glucose déshydrogénase (FAD-GDH, 11580 Unité/mg solide (*une unité d'activité : quantité d'enzyme qui va convertir une micromole de glucose (β-Dextrose) par minute à 37°C.*)) *d'aspergillus sp.* (Avril 2018 : No.EC 1.1.5.9) provient de la société SEKISUI.

Les nanotubes de carbone à parois multiples (MWCNT) ont été achetés auprès de la société Nanocyl (pureté> 95%, diamètre 10 nm, longueur 1,5 µm).

Le noir de carbone de marque VULCAN^{®} XC72R a été acheté à la société CABOT corporation.

La couche de diffusion de gaz microporeuse (GDL) collectrice de courant a été achetée auprès de la société Paxitech. Elle est constituée d'un maillage étroit de fibres de carbone sous forme d'un feutre de carbone et présente une épaisseur de 210 µm, une résistivité traversante de 8 mΩ cm² et une perméabilité traversante à l'air de 70 secondes.

Le papier poreux utilisé en tant que coussinet est un papier buvard super-épais (grade 707) acheté chez VWR de référence 732-0604. Il s'agit d'un papier buvard 100% coton, sans additifs, et fabriqué avec de l'eau Ultrapure. Sa texture est lisse et régulière et sa structure homogène. Sa masse surfacique est de 703 g/m² pour une épaisseur de 2.6 mm. Ses caractéristiques techniques données sont un temps de filtration (selon Herzberg) de 120s/100 ml (avec une colonne d'eau de 100 mm) ainsi qu'une montée de 75 mm en 96 secondes selon le test de Klemm (taux de pénétration capillaire).

La feuille de cellulose d'épaisseur 140 µm utilisée est de type Whatman (*qualitative filter paper for technical use, Grade 0903)* distribué par Sigma Aldrich (réf WHA10334885).

Le film de protection est une feuille adhésive d'un tissu ou film de verre imprégné de PTFE (TISOFLON 3 AD ; société ISOFLON) d'épaisseur 70 µm et ayant un taux d'imprégnation en PTFE de 61% en poids. La surface adhésive de ce film est recouverte d'une feuille protectrice en PTFE (Teflon^{™}). Ce film est recouvert d'une feuille protective en téflon qui peut servir de cadre de maintien ou gabarit aux pastilles bio-anodiques et/ou bio-cathodiques selon l'invention.

Les enzymes ont été stockées à -20 " C. L'eau distillée a été obtenue par purification de l'eau à une résistivité de 15 MΩ × cm en utilisant un système Millipore Ultrapure^{™}. L'oxygène et l'argon de haute pureté provient de la société MESSER. Les solutions de glucose ont été laissées à se transformer en une nuit en β-D-glucose avant utilisation. Tous les autres réactifs, y inclut la 1,4-naphtoquinone proviennent de la Société Sigma-Aldrich Inc.

Pour la caractérisation électrochimique de la cellule de biocombustible, l'anode a été définie comme l'électrode de travail tandis que la cathode a été connectée en tant qu'électrode de référence. Toutes les expériences ont été réalisées avec une solution de glucose de 5 (proche de la concentration de certains liquides biologiques) ou 150 mmol L-1 dans Mcllvaine ou un tampon phosphate, à pH 7. La solution a été déposée au compte-goutte sur la tranche du réservoir du moins sur une qui n'est pas totalement recouverte de revêtement isolant. La cellule de biocombustible a été connectée à un potentiostat multicanaux Biologic^{®} VMP3 exécutant le logiciel EC-lab 10.39. Les courbes de polarisation et de puissance ont été enregistrées après une décharge de 30 secondes. Toutes les expériences ont été réalisées à température ambiante (env. 20°C).

Les biocathodes et bioanodes exemplifiés ci-dessous sont des solides façonnés sous forme de disques comprimés.

### Exemple 1 (pastilles insérées dans une feuille poreuse)

Pour la biocathode, les nanotubes de carbone (MWCNT) ont été mélangés à la protoporphyrine IX en présence de DMF (Dimethylformamide) puis chauffé à 80°C pendant environ 12 heures. La protoporphyrine IX favorise l'orientation des protéines enzymatiques par rapport aux nanotubes. Les nanotubes ainsi fonctionnalisés ont été rincés et filtrés plusieurs fois à l'eau distillée. 35 mg de MWCNT fonctionnalisés par de la protoporphyrine IX ont été broyés en présence de 15 mg de BOD et de 100 µl d'eau distillée.

Pour une bioanode, 35 mg de MWCNT, 15 mg de GOx, 10 mg de catalase provenant du foie de bovin et 5 mg de 1,4-naphtoquinone ont été broyés avec 100 µL d'eau distillée. Pour une autre bioanode à base de FAD-GDH, 35 mg de MWCNT fonctionnalisés, 15 mg de FAD-GDH et 5 mg de 1,4-naphtoquinone ont été broyés avec 100 µL d'eau distillée.

Chaque pâte homogène est ensuite comprimée respectivement sur une languette GDL de contact électrique à l'aide d'une presse hydraulique pour obtenir une pastille de 1 cm de diamètre et de 2 mm d'épaisseur. Dans ce cas, cette languette de GDL 9 ou 9' sert à la fois de collecteur de courant et de support pour la compression de la pâte de carbone.

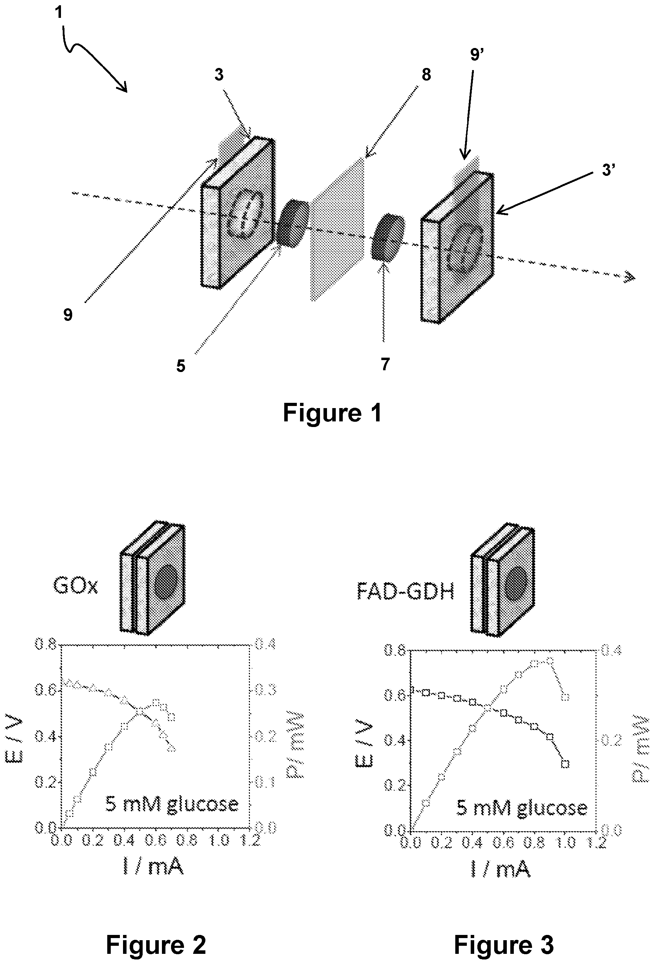

Des trous de dimensions et de formes correspondantes à celles des pastilles anodique et cathodique sont effectués dans un coussinet (une feuille de papier buvard) de 2.6 mm d'épaisseur et de dimension de 25mm × 25 mm. Chaque coussinet, ou « pad », va servir de cadre à une pastille. Le dispositif de la biopile est alors assemblé : chacune des associations pastille/languette réalisées est positionnés à l'intérieur des ouvertures des coussinets par l'arrière de ceux-ci de manière à positionner les languettes de GDL à l'extérieur et en saillie par rapport à leur coussinet respectif. Une bioanode et une biocathode sont ainsi constituées. Une fine feuille de cellulose Whatman servant d'isolant électrique est placée entre la bioanode et la biocathode. Cet assemblage constitue la base de la cellule électrochimique. La figure 1 est une vue semi-éclatée d'un tel dispositif. Une pastille enzymatique 5, ou bioanode, comprenant de la GOx ou de la FAD-GDH, est positionnée dans le réservoir à combustible 3, la languette de GDL 9 faisant saillie à l'arrière. De manière symétrique, une pastille enzymatique 7, ou biocathode, comprenant de la BOD est positionnée dans un coussinet 3', la languette de GDL 9' faisant également saillie à l'arrière de celui-ci. La feuille poreuse d'isolant électrique 8 est placée entre la bioanode 5 et la biocathode 7 et leur cadre respectif 3 et 3'.

Ce dispositif est alors enveloppé par un film protecteur adhésif en tissu de verre et PTFE d'épaisseur 70 µm (non représenté) qui recouvre l'arrière des deux électrodes. Les portions des languettes de GDL en saillie ne sont pas substantiellement recouvertes de ce film protecteur. De plus, 4 trous de 2 mm de diamètre effectués dans le film protecteur sont positionnés à l'arrière de la biocathode et permettent la diffusion de l'oxygène de l'air à l'intérieur du dispositif et sa mise à disposition à la biocathode 7.

Les figures 2 et 3 représentent les courbes de polarisation et de puissance des Biopiles GOx (Figure 2) et FAD-GDH (Figure 3). Les puissances obtenues dans le cas de la FAD-GDH (0.38 mW) sont supérieurs aux puissances obtenues dans le cas de la GOx (0.27 mW). Ceci vient du fait que la FAD-GDH est plus active que la GOx et qu'elle ne génère pas de H₂O₂ en tant que coproduit. En effet, H₂O₂ peut augmenter l'instabilité de la pile et inhiber l'activité de l'enzyme BOD à la cathode.

### Exemple 2 : Empilement (« stack »)

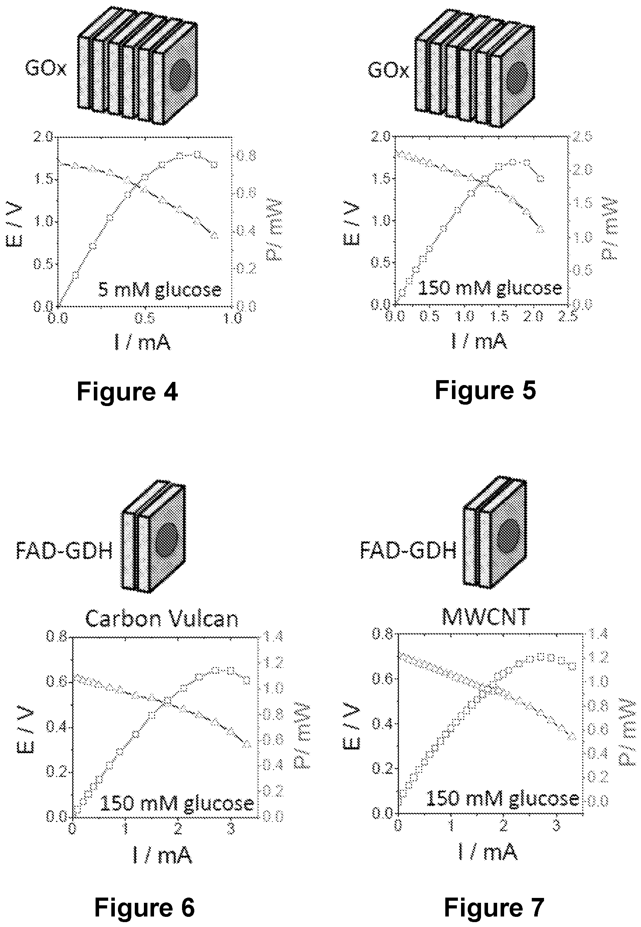

Un stack/empilement de 3 cellules avec une biocathode/anode FAD-GOX/BOD est représenté de manière schématique (languettes de GDL non représentées) à la Figure 3 et à la figure 4. Cet empilement été réalisé en ne laissant que les languettes extérieures faire saillie de manière à fermer le circuit. Une fois les cellules juxtaposées, l'empilement a été recouvert d'un film adhésif en tissu de fibres de verre et de PTFE de la même manière que le dispositif de l'exemple 1.

Cet exemple montre la facilité d'empilement de ces piles, passant de 0,27 mW et 0,62 V de f.e.m. pour une pile seule à 0,82 mW et 1,7 V de f.e.m. pour un empilement de 3 piles avec un coussinet imbibé d'une solution à 5 mM de glucose (Figure 4). Une solution concentrée de glucose (150 mM) donne accès à des puissances élevées de 2.2 mW (Figure 5). Les figures 4 et 5 montrent l'effet de ces deux concentrations de glucose différentes sur les performances obtenues.

### Exemple 3 : Biopile FAD-GDH/BOD utilisant un support en noir de carbone

Afin de s'affranchir des problèmes qui peuvent être liés aux nanotubes de carbone en terme d'accès à des échantillons commerciaux ou de par leur suspicion de toxicité, les MWCNTs ont été remplacés par du noir de carbone, déjà utilisé dans de nombreux produits commerciaux.

Des pastilles enzymatiques sont obtenues en utilisant le même procédé que l'exemple 1 mais en remplaçant les MWCNT par du noir de carbone (Vulcan ^{®}). Une biopile à 1 cellule est alors réalisée en utilisant également le procédé de l'exemple 1. La Figure 6 représente les courbes de polarisation et de puissance pour cette biopile FAD-GDH sur Carbone Vulcan^{®} obtenue lorsque mise en présence avec une solution de glucose de 150 mM.

La Figure 7 représente les courbes de polarisation et de puissance de la biopile FAD-GDH sur MWCNT de l'exemple 1 lorsque mise en présence avec une solution de glucose de même concentration (150 mM). Les puissances délivrées par des piles à base de MWCNTs et de carbone Vulcan (noir de carbone) sont respectivement de 1.3 et 1.2 mW pour une recharge en glucose de 150 mM (dans environ 0.5 mL). Les deux matériaux donnent accès à des puissances similaires et peuvent donc être utilisés tous les deux en fonction des contraintes techniques et environnementales nécessaires à la fabrication de ces piles.

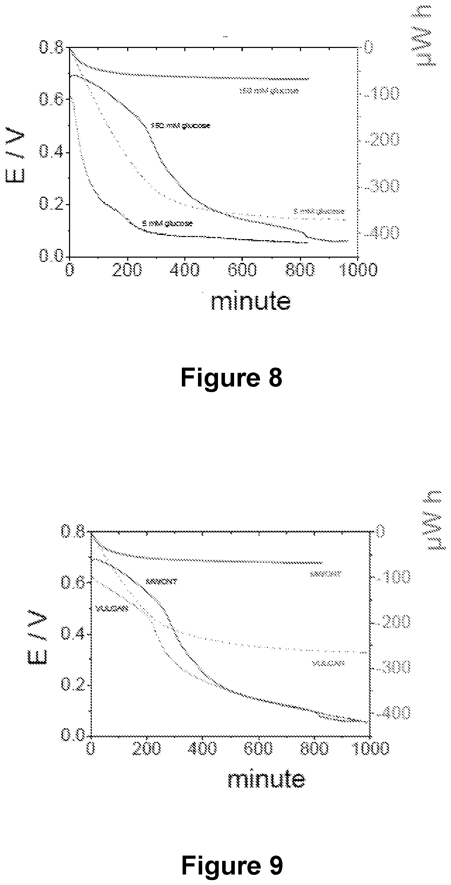

La Figure 8 montre la production d'énergie par décharge continue à 6 kΩ en µW.h par rapport au temps (minutes) et consommation totale du glucose pour des coussinets imbibés de glucose à 5mM et 150 mM pour la biopile de l'exemple 3 (noir de carbone). La figure 9 montre la production d'énergie par décharge continue à 6 kΩ en µWh par rapport au temps (minutes) et consommation totale du glucose pour des coussinets imbibés de glucose à 150 mM pour la biopile de l'exemple 3 (noir de carbone) et celle de l'exemple 1 FAD-GDH/BOD sur MWCNTs.

Les meilleures performances sont obtenues pour la pile à base de MWCNTs qui permet de générer 400 µWh après une décharge en continu qui consomme la totalité des 150 mM de glucose disponible.

### Exemple 4 : Biopile à réservoir unique

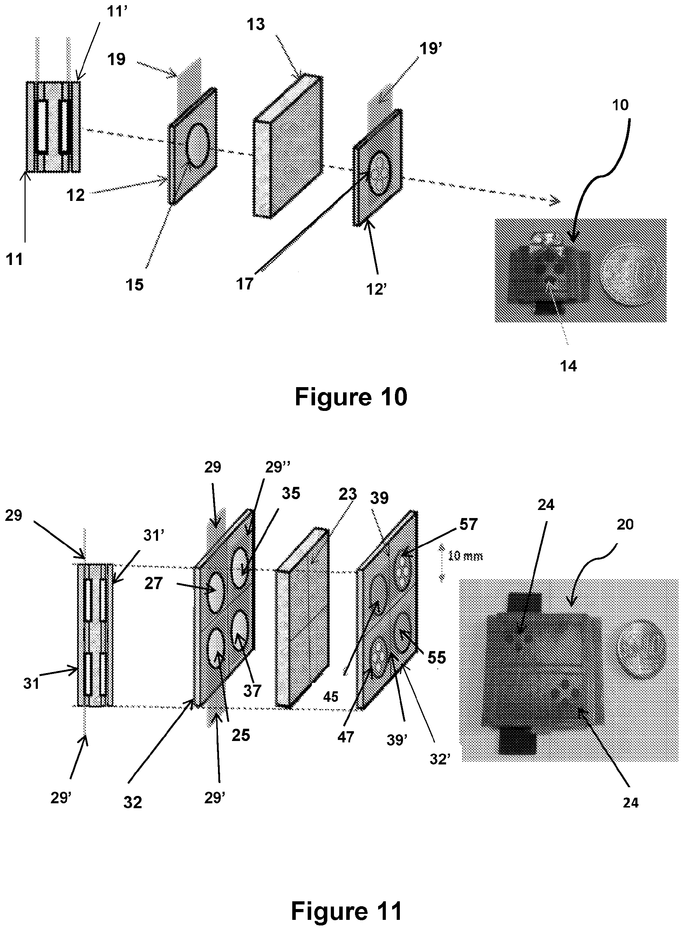

La figure 10 comprend un schéma de la structure d'une biopile 10 selon cet exemple particulier.

Pour la biocathode, 8,75 mg de MWCNT fonctionnalisés par la protoporphyrine IX tels que décrits à l'exemple 1 ont été broyés en présence de 3,75 mg de BOD et de 25 µl d'eau distillée. Pour la bioanode, 8,75 mg de MWCNT, 3,75 mg de GOx, 2,5 mg de catalase provenant du foie de bovin et 1,25 mg de 1,4-naphtoquinone ont été broyés avec 25 µL d'eau distillée. Chaque pastille 15 et 17 est formée in-situ sur un cadre qui comprend une feuille en PTFE 12 ou 12' ayant une épaisseur de 0,25 mm et percé d'un trou circulaire d'1 cm de diamètre. Ce gabarit est placé sur un support constitué d'une feuille adhésive en fibre de verre et PTFE 11 ou 11' d'épaisseur 70 µm. Une languette en GDL 19 ou 19' est positionnée vis-à-vis du trou circulaire de la feuille en PTFE 12 ou 12' entre le trou circulaire et la feuille adhésive 11 ou 11'. Cette feuille 11 et 11' n'est pas représentée sur la vue éclatée. Une quantité suffisante de chaque pâte homogène de bioanode ou de biocathode (une quantité d'environ 12 mg par pastille permet de remplir un gabarit) est positionnée dans le trou et est comprimée directement sur le support par une presse. Ceci permet d'obtenir des pastilles de 1 cm de diamètre et de 0,25 mm d'épaisseur. Ces pastilles sur support formées *in-situ* peuvent alors être utilisées comme bioanodes et biocathodes.

La feuille adhésive 11' de la biocathode est percée de 4 trous 14 de 2 mm de diamètre pour permettre la diffusion de l'oxygène de l'air à la biocathode. Bien que la feuille adhésive de revêtement 11 ou 11' ne soit pas représentée sur la vue éclatée de la figure 10, les positions qu'adopteraient les trous y sont indiquées. Les dimensions de la feuille de PTFE servant de cadre 12 ou 12' est d'environ 20mm × 20mm. Les feuilles de support externe 11 et 11' qui servent de protection sont de dimensions plus étendues et permettent par contact de leurs surfaces adhésives en regard de solidariser ou joindre les éléments du dispositif. Leurs faces adhésives étant face à face, elles peuvent être jointes l'une à l'autre et solidariser la biopile sur certains de ses cotés. Un exemple de cette variante fait l'objet d'une photographie encadrée en Figure 10. On peut y voir la feuille externe flexible 11' percée des trous 14 et s'étendant latéralement de droite et de gauche pour recouvrir et adhérer aux parties symétriques de la feuille 11 qui se recouvrent l'une l'autre. Une pièce de 10 centimes d'euro permet de visualiser la dimension de la biopile obtenue.

Une feuille de buvard épais 13 (tel qu'utilisé aux exemples précédents) sensiblement de même dimension que la feuille de PTFE servant de cadre est alors placée entre la bioanode 15 et la biocathode 17. Les languettes de GDL 19 et 19' servent à la fois de collecteur de courant et de support pour les pastilles 15 ou 17.

Une languette de GDL 19 est donc positionnée en regard de la pastille anodique 15, et une languette de GDL 19' est également positionnée en regard de la pastille cathodique 17. Ces deux languettes 19 et 19' font saillie par rapport à leur support respectif 12 et 12'. Elles sont positionnées entre le cadre en PTFE 12 ou 12' et la feuille externe de support 11 ou 11'. Ces languettes 19 et 19' servent à la fois de collecteur de courant et de support pour la pastille qui leur est associée et permet de connecter le dispositif à un récepteur électrique. Dans la vue de face de la biopile 10 les languettes 19 et 19' font saillie sur des cotés opposées du dispositif selon l'invention alors que sur la vue éclatée ces languettes 19 et 19' sont représentées se projetant d'un même côté. Les deux dispositions sont possibles.

Quelle que soit la forme particulière finale d'une biopile selon l'invention, elle comprend un passage permettant l'accès du combustible à base de glucose vers le coussinet 13. Ici la solution de glucose est ajoutée en injectant une solution de glucose à l'aide d'une seringue.

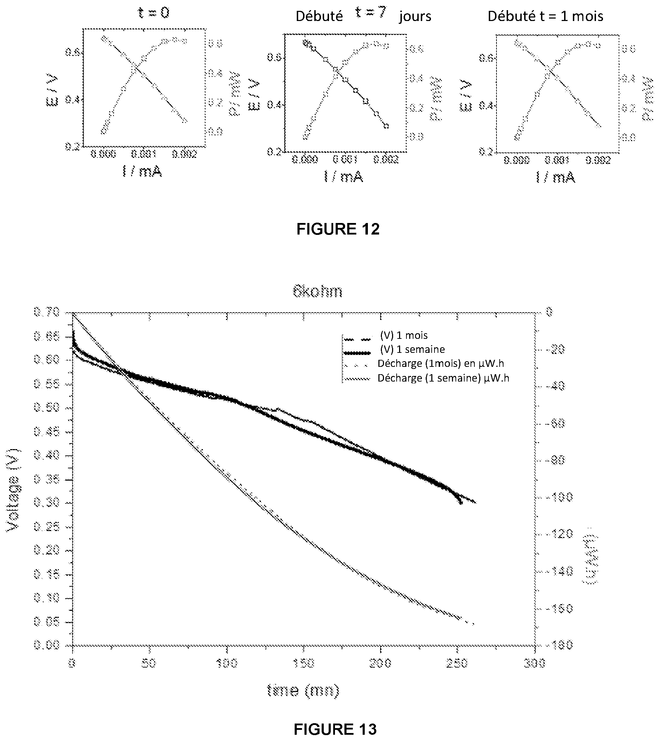

La Figure 12 montre que la biopile garde ses performances que celle-ci soient mesurées après une semaine ou 1 mois. La figure 13 confirme qu'en testant la décharge de la biopile, celle-ci conserve également 100% de ses performances qu'elle soit démarré après une semaine ou un mois. Ceci valide la capacité de la biopile à alimenter un dispositif au moins 1 mois après sa fabrication sans perte de performances.

### Exemple 5 biopiles à pastilles en série

La figure 11 comprend un schéma de la structure d'une biopile 20 selon cet exemple particulier.

Les pastilles anodiques et cathodiques utilisées sont de même type que celles utilisées pour le dispositif de l'exemple 4.

Quatre pastilles, deux anodiques 25 et 35, et deux cathodiques 27 et 37 sont disposées sur la surface adhésive d'une première feuille adhésive de fibre de verre et de PTFE 31 et dans quatre ouvertures circulaires d'un cadre de PTFE 32 de dimensions 35 mm x 35 mm. Ces feuilles sont de matériaux et d'épaisseurs identiques à ceux décrits à l'exemple 4. Les quatre pastilles sont positionnées selon un quadrilatère et chaque pastille anodique 25 et 35 est disposée à la diagonale l'une de l'autre ainsi que les pastilles cathodiques 27 et 37.

Une autre série de quatre pastilles dans un cadre de PTFE 32'sont disposées sur une seconde feuille en fibre et PTFE 31'. Sur le second cadre 32', les 4 pastilles sont alternées selon une disposition symétrique : deux pastilles anodiques 45 et 55, et deux cathodiques 47 et 57 chacune à la diagonale de l'autre.

La mise en série des cellules électrochimiques est réalisée par l'utilisation de languettes de GDL 29, 29', et 29" positionnées entre la feuille externe adhésive 31 et le cadre en PTFE 32 et des languettes 39 et 39' positionnées entre la feuille externe adhésive 31' et le cadres en PTFE 32'. Il convient de noter que sur le schéma éclaté de la Figure 11 la position des languettes sur le cadre en PTFE 32 est vue par transparence : les languettes sont en fait positionnées derrière la face du cadre représenté. Ceci est fait pour pouvoir apprécier le positionnement respectif des languettes. Leur position relative est mieux explicitée dans la vue de coupe associée. Sur le cadre de PTFE 32', la languette 39 positionnée horizontalement entre les pastilles 45 et 57 permet de connecter la cellule électrochimique constituée de l'anode 45 et la cathode 27 à celle constituée de l'anode 35 et de la cathode 57. De même, la languette 29" positionnée verticalement entre les pastilles 35 et 37 permet de connecter la cellule constituée de l'anode 35 et la cathode 57 à celle constituée de l'anode 55 et de la cathode 37. La languette 39' positionnée horizontalement entre les pastilles 55 et 47 permet de connecter la cellule constituée de l'anode 55 et la cathode 37 à celle constituée de l'anode 25 et de la cathode 47. Les languettes 29 et 29', respectivement en contact avec la cathode 27 et l'anode 25 se projettent à l'extérieur du circuit dans des directions opposées et permettent la connexion du dispositif à un récepteur électrique (non représenté).

Comme pour l'exemple 4, les pastilles sont formées par compression in situ par ajout d'une quantité de pâte dans les trous circulaire de 10 mm de diamètre préformés dans le cadre 32 ou 32'. Les languettes 29, 29', 29", 39 et 39' sont positionnées au préalable entre la feuille de fibre de verre et de PTFE 31 et 31' servant de support et le cadre en PTFE 32 et 32'.

Des séries de 4 trous 24 de 2 mm de diamètre sont réalisés sur les feuilles de support en fibre de verre et PTFE 31 et 31' à l'arrière de chacune des 4 biocathodes 27, 37, 47 et 57 (et de la portion de languettes GDL correspondantes) et permettent la diffusion de l'oxygène de l'air à la biocathode. La position de ces séries d'ouvertures 24 est également indiquée sur la vue partielle éclatée du dispositif 20 bien que les ouvertures soient effectuées dans les feuilles 31 et 31', non représentées sur la vue partielle éclatée.

Un réservoir, ou pad, de 3mm d'épaisseur et de substantiellement même dimension, ou légèrement inférieure, que celle des cadres en PTFE 32 et 32' est interposé entre les deux feuilles portant les cadres en PTFE 31 et 31', les pastilles 27, 35, 37, 25 et 45, 57, 55, 47, et portant les languettes 29, 29' et 29". Ce pad 23 est directement en contact avec une face des pastilles 27, 35, 37, 25, 45, 57, 55 et 47. Ce pad est en buvard épais de même type que celui décrit précédemment à l'exemple 4.

Les feuilles de revêtement externe 31 et 31' qui servent de protection et de support sont de dimensions plus étendues que le cadre en PTFE et se projettent donc au delà de ces cadres 32 et 32'. Ainsi ces feuilles 31 et 31' permettent, par contact de leurs surfaces adhésives en regard de solidariser, ou joindre, les éléments du dispositif. Leurs faces adhésives étant en regard, elles peuvent être jointes l'une à l'autre et solidariser la biopile sur certains de ses côtés. Un exemple de cette variante fait l'objet de la vue de face de la figure 20. On peut y voir la feuille externe flexible 31' percée des séries de trous 14 dont les bords s'étendent latéralement à droite et à gauche pour recouvrir et adhérer aux parties symétriques de la feuille 31 en se recouvrant l'une l'autre.

Les cadres 32 et 32' sont mis face à face de manière à ce que chaque pastille anodique 25, 35 placée sur une première feuille 32 soit en regard d'une pastille cathodique 47 et 57 placée sur l'autre feuille en PTFE 32. De même, chaque pastille anodique 27, 37 placée sur la première feuille en PTFE 32 est en regard d'une pastille cathodique 45 et 55 placée sur l'autre feuille en PTFE 32'. Le réservoir 23, en feuille de buvard épais de même dimension que les feuilles de PTFE 32 et 32' est alors placé entre celles-ci. Le dispositif 30 est alors obtenu en contactant les feuilles de revêtements externes 31 et 31' l'une à l'autre.

### Exemple 6: Comparaison des biopiles à pastilles en série de l'exemple précédent avec une pastille unique

Une biopile 40 selon l'invention a été réalisée pour comparer l'efficacité du dispositif en série décrit à l'exemple 5 avec un biopile de même type mais ne comprenant qu'un couple de pastilles, et non 4, montées en série, à quantité de matériau égale. Une biopile comprenant un couple de pastilles bio-anodique et bio-cathodique circulaires, identiques à l'exemple 5 en tous points, à l'exception de leurs diamètres, a été réalisé. Le diamètre des pastilles est de 20 mm.

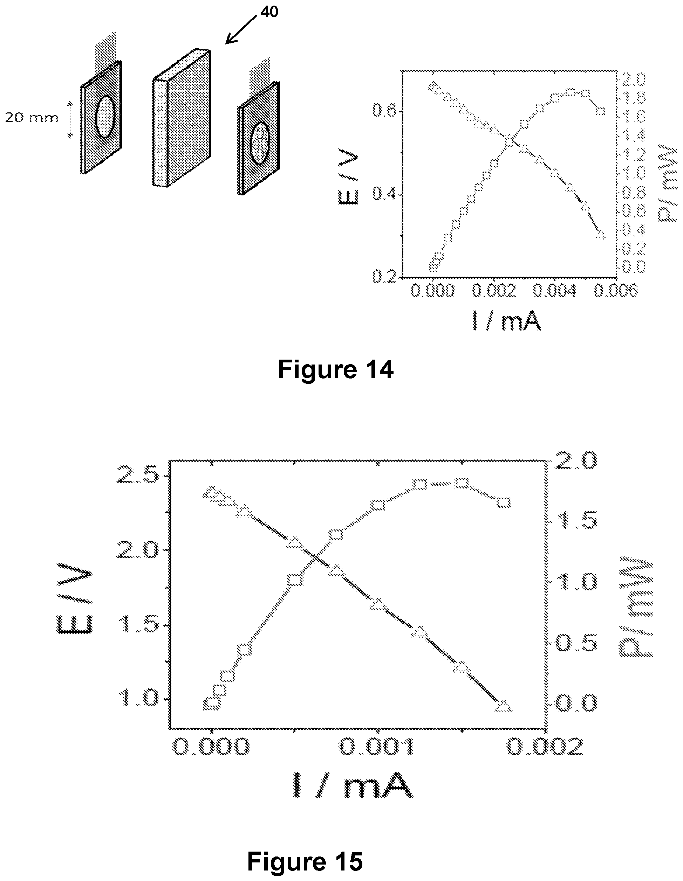

La Figure 14 représente les courbes de polarisation et de puissance de la biopile à pastille de 20mm. La figure montre que si on augmente la taille des pastilles par deux, on obtient une puissance multipliée par deux approximativement. La Figure 15 représente les courbes de polarisation et de puissance de la biopile décrite à l'exemple 5. Elle montre que si deux biopiles sont mises en série dans le même dispositif, on augmente la f.e.m et la puissance de la pile par deux.

## Revendications

1. Une biopile (1, 10, 20) à réservoir de biocombustible, ladite biopile (1, 10, 20) étant destinée à être mise en contact avec un milieu liquide, ledit milieu liquide comprenant éventuellement un biocombustible, et avec un milieu fluide comprenant un oxydant, ladite biopile comprenant une première cellule électrochimique, ladite première cellule électrochimique comprenant :
- une anode (5, 15, 25, 35, 45, 55) constituée d'un agglomérat solide comprenant un matériau conducteur mélangé à une première enzyme apte à catalyser l'oxydation du biocombustible ; et
- une cathode (7, 17, 27, 37, 47, 57) constituée d'un agglomérat solide comprenant un matériau conducteur mélangé à une seconde enzyme apte à catalyser la réduction de l'oxydant, et
- une membrane séparatrice et poreuse (3, 3', 8, 13, 23), électriquement isolante, et perméable audit milieu liquide, placée entre l'anode (5, 15, 25, 35, 45, 55) et la cathode (7, 17, 27, 37, 47, 57),
- ladite biopile comprenant, en outre, des moyens de mise en circuit électrique (9, 9', 19, 19', 29, 29', 29", 39, 39') de ladite biopile avec un récepteur électrique, lesdits moyens de mise en circuit électrique permettant la circulation d'un courant de la cathode (7, 17, 27, 37, 47, 57) vers l'anode (5, 15, 25, 35, 45, 55);
ladite biopile (1, 10, 20) étant **caractérisée en ce qu'**elle comprend un moyen de stockage du biocombustible (3, 3', 13, 23) et de mise à disposition du milieu liquide à l'anode (5, 15, 25, 35, 45 et 55), ledit moyen comprenant un matériau poreux hydrophile en contact avec ladite anode (5, 15, 25, 35, 45 et 55) et présentant une masse surfacique de 500 à 900 g/m².

2. La biopile (1, 10, 30) selon la revendication 1, où ledit moyen de stockage du biocombustible (3, 3', 13, 23) à une épaisseur de 1 cm à 0,1 mm.

3. La biopile (1, 10, 30) selon la revendication 1 ou 2, où ledit matériau poreux hydrophile est à base de cellulose.

4. La biopile (1, 10, 30) selon l'une quelconque des revendications 1 à 3, où ledit moyen de stockage du biocombustible et de mise à disposition du liquide (13 et 23) est également ladite membrane (13 et 23) séparatrice et poreuse, électriquement isolante, et perméable au milieu liquide.

5. La biopile (1, 10, 30) selon l'une quelconque des revendications 1 à 4, où lesdits moyens de mise en circuit du dispositif comprennent une languette en graphite pyrolytique.

6. La biopile (1, 10, 30) selon l'une quelconque des revendications 1 à 5, où ladite anode (5, 15, 25, 35, 45 et 55) est sous forme de pastille.

7. La biopile (1, 10, 30) selon l'une quelconque des revendications 1 à 6, où ladite anode est maintenue dans un cadre, ledit cadre étant soit ledit moyen de stockage du biocombustible et de mise à disposition du milieu liquide (3, 3',13 et 23), soit un matériau électriquement isolant tel que du PTFE.

8. La biopile (1, 10, 30) selon l'une quelconque des revendications 1 à 7, ou ladite biopile comprend un revêtement externe, de préférence flexible et isolant, comprenant des ouvertures positionnées et dimensionnées de manière à permettre l'accès d'un liquide à l'anode et/ou d'un gaz à la cathode.

9. La biopile (1, 10, 30) selon l'une quelconque des revendications précédentes et comprenant, en outre, un milieu liquide aqueux, ledit liquide comprenant éventuellement un biocombustible.

10. Utilisation d'une biopile selon l'une quelconque des revendications 1 à 9, pour la génération d'un courant électrique.

## Patentansprüche

1. Biobrennstoffzelle (1, 10, 20) mit einem Biobrennstoffspeicher, wobei die Biobrennstoffzelle (1, 10, 20) dazu bestimmt ist, mit einem flüssigen Medium, das gegebenenfalls einen Biobrennstoff umfasst, und mit einem flüssigen Medium, das ein Oxidationsmittel umfasst, in Kontakt gebracht zu werden, wobei die Biobrennstoffzelle eine erste elektrochemische Zelle umfasst, wobei die erste elektrochemische Zelle umfasst:
- eine Anode (5, 15, 25, 35, 45, 55), die aus einem festen Agglomerat besteht, das ein leitfähiges Material enthält, das mit einem ersten Enzym vermischt ist, das in der Lage ist, die Oxidation des Biobrennstoffs zu katalysieren; und
- eine Kathode (7, 17, 27, 37, 47, 57), die aus einem festen Agglomerat besteht, das ein leitfähiges Material enthält, das mit einem zweiten Enzym vermischt ist, das in der Lage ist, die Reduktion des Oxidationsmittels zu katalysieren, und
- eine poröse Trennmembran (3, 3', 8, 13, 23), die elektrisch isolierend und für das flüssige Medium durchlässig ist und zwischen der Anode (5, 15, 25, 35, 45, 55) und der Kathode (7, 17, 27, 37, 47, 57) angeordnet ist,
- wobei die Biobrennstoffzelle ferner Mittel zum elektrischen Verschalten (9, 9', 19, 19', 29, 29', 29", 39, 39') der Biobrennstoffzelle mit einem elektrischen Verbraucher umfasst, wobei die Mittel zum elektrischen Verschalten das Fließen eines Stroms von der Kathode (7, 17, 27, 37, 47, 57) zu der Anode (5, 15, 25, 35, 45, 55) ermöglichen;
wobei die Biobrennstoffzelle (1, 10, 20) **dadurch gekennzeichnet ist, dass** sie ein Mittel zum Speichern des Biobrennstoffs (3, 3', 13, 23) und zum Zuführen des flüssigen Mediums zu der Anode (5, 15, 25, 35, 45 und 55) umfasst, wobei das Mittel ein hydrophiles poröses Material in Kontakt mit der Anode (5, 15, 25, 35, 45 und 55) umfasst und ein Flächengewicht von 500 bis 900 g/m² aufweist.

2. Biobrennstoffzelle (1, 10, 30) nach Anspruch 1, wobei das Mittel zum Speichern des Biobrennstoffs (3, 3', 13, 23) eine Dicke von 1 cm bis 0,1 mm hat.

3. Biobrennstoffzelle (1, 10, 30) nach Anspruch 1 oder 2, wobei das hydrophile poröse Material auf Zellulose basiert.

4. Biobrennstoffzelle (1, 10, 30) nach einem der Ansprüche 1 bis 3, wobei das Mittel zum Speichern des Biobrennstoffs und zum Zuführen der Flüssigkeit (13 und 23) auch die poröse Trennmembran (13 und 23) ist, die elektrisch isolierend und für das flüssige Medium durchlässig ist.

5. Biobrennstoffzelle (1, 10, 30) nach einem der Ansprüche 1 bis 4, wobei die Mittel zum elektrischen Verschalten der Vorrichtung eine Lasche aus pyrolytischem Graphit umfassen.

6. Biobrennstoffzelle (1, 10, 30) nach einem der Ansprüche 1 bis 5, wobei die Anode (5, 15, 25, 35, 45 und 55) in Form einer Pastille vorliegt.

7. Biobrennstoffzelle (1, 10, 30) nach einem der Ansprüche 1 bis 6, wobei die Anode in einem Rahmen gehalten wird, wobei der Rahmen entweder das Mittel zum Speichern des Biobrennstoffs und zum Zuführen des flüssigen Mediums (3, 3', 13 und 23) oder ein elektrisch isolierendes Material wie PTFE ist.

8. Biobrennstoffzelle (1, 10, 30) nach einem der Ansprüche 1 bis 7, wobei die Biobrennstoffzelle eine vorzugsweise flexible und isolierende Außenbeschichtung umfasst, die Öffnungen umfasst, die so positioniert und dimensioniert sind, dass sie den Zugang einer Flüssigkeit zu der Anode und/oder eines Gases zu der Kathode ermöglichen.

9. Biobrennstoffzelle (1, 10, 30) nach einem der vorhergehenden Ansprüche und ferner umfassend ein wässriges flüssiges Medium, wobei die Flüssigkeit gegebenenfalls einen Biobrennstoff umfasst.

10. Verwendung einer Biobrennstoffzelle nach einem der Ansprüche 1 bis 9 zur Erzeugung von elektrischem Strom.

## Claims

1. A biocell (1, 10, 20) having a biofuel reservoir, said biocell (1, 10, 20) being intended to be brought into contact with a liquid medium, said liquid medium optionally comprising a biofuel, and with a fluid medium comprising an oxidizer, said biocell comprising a first electrochemical cell, said first electrochemical cell comprising:
- an anode (5, 15, 25, 35, 45, 55) made of a solid agglomerate comprising a conductive material mixed with a first enzyme capable of catalyzing the oxidation of the biofuel; and
- a cathode (7, 17, 27, 37, 47, 57) made of a solid agglomerate comprising a conductive material mixed with a second enzyme capable of catalyzing the reduction of the oxidant, and
- a separating and porous membrane (3, 3', 8, 13, 23) which is electrically insulating and permeable to said liquid medium and is placed between the anode (5, 15, 25, 35, 45, 55) and the cathode (7, 17, 27, 37, 47, 57),
- said biocell further comprising means for placing said biocell in an electrical circuit (9, 9', 19, 19', 29, 29', 29", 39, 39') with an electric receiver, said means for placing in an electrical circuit allowing a current to flow from the cathode (7, 17, 27, 37, 47, 57) to the anode (5, 15, 25, 35, 45, 55),
said biocell (1, 10, 20) being **characterized in that** it comprises a means for storing the biofuel (3, 3', 13, 23) and for providing the liquid medium to the anode (5, 15, 25, 35, 45 and 55), said means comprising a hydrophilic porous material in contact with said anode (5, 15, 25, 35, 45 and 55) and having a mass per unit area of 500 to 900 g/m².

2. The biocell (1, 10, 30) according to claim 1, wherein said means (3, 3', 13, 23) for storing the biofuel has a thickness of 1 cm to 0.1 mm.

3. The biocell (1, 10, 30) according to either claim 1 or claim 2, wherein said hydrophilic porous material is cellulose-based.

4. The biocell (1, 10, 30) according to any one of claims 1 to 3, wherein said means for storing the biofuel and for providing the liquid (13 and 23) is also said separating and porous membrane (13 and 23) which is electrically insulating and permeable to the liquid medium.

5. The biocell (1, 10, 30) according to any one of claims 1 to 4, wherein said means for placing the device in a circuit comprise a tab of pyrolytic graphite.

6. The biocell (1, 10, 30) according to any one of claims 1 to 5, wherein said anode (5, 15, 25, 35, 45 and 55) is in the form of a pellet.

7. The biocell (1, 10, 30) according to any one of claims 1 to 6, wherein said anode is held in a frame, said frame being either said means for storing the biofuel and for providing the liquid medium (3, 3', 13 and 23), or an electrically insulating material such as PTFE.

8. The biocell (1, 10, 30) according to any one of claims 1 to 7, wherein said biocell comprises an outer covering, which is preferably flexible and insulating, comprising openings that are positioned and dimensioned so as to allow a liquid to enter the anode and/or gas to enter the cathode.

9. The biocell (1, 10, 30) according to any one of the preceding claims and further comprising an aqueous liquid medium, said liquid optionally comprising a biofuel.

10. Use of a biocell according to any one of claims 1 to 9, for generating an electric current.
